# EUROPÄISCHE PATENTSCHRIFT

(11) **EP 1 976 925 B1**
(45) Veröffentlichungstag und Bekanntmachung des Hinweises auf die Patenterteilung: **27.05.2009**
(21) Anmeldenummer: 07703822.2
(22) Anmeldetag: 12.01.2007
(51) Int. Cl.: C08K 5/3435, C08K 5/00

(54) **STABILISATORMISCHUNG**
STABILIZER MIXTURE
MELANGE STABILISATEUR

(30) Priorität: 13.01.2006 EP 06100343
(43) Veröffentlichungstag der Anmeldung: 08.10.2008
(73) Patentinhaber: BASF SE, 67056 Ludwigshafen (DE)
(72) Erfinder: SCHAMBONY, Simon, 67067 Ludwigshafen (DE); GLASER, Alban, 68159 Mannheim (DE); TRAUTH, Hubert, 67373 Dudenhofen (DE); APPEL, Manfred, 76857 Dernbach (DE); HAREMZA, Sylke, 69151 Neckargemünd (DE)
(74) Vertreter: Isenbruck, Günter
(86) Internationale Anmeldenummer: PCT/EP2007/050281
(87) Internationale Veröffentlichungsnummer: WO 2007/082842

(56) Entgegenhaltungen:
- EP-A1- 1 413 599
- WO-A-02/092684
- DE-A1- 4 239 437
- US-A1- 2004 030 009

## Beschreibung

Die Erfindung betrifft eine Mischung aus oligomeren und monomeren Verbindungen vom Typ HALS, ein Verfahren zu deren Herstellung sowie deren Verwendung als Stabilisatoren für organisches Material.

Organisches Material, insbesondere Kunststoffe und Lacke, wird-bekanntermaßen sehr schnell, vor allem durch Einwirkung von Licht, zerstört. Diese Zerstörung zeigt sich üblicherweise in Vergilbung, Verfärbung, Rissbildung oder Versprödung des Materials. Mit Lichtschutzmitteln und Stabilisatoren soll daher ein zufriedenstellender Schutz gegen die Zerstörung von organischem Material durch Licht, Sauerstoff und Wärme erzielt werden.

Derivate des 2,2,6,6-Tetraalkylpiperidins sind unter der Bezeichnung HALS (Hindered Amine Light Stabilizers) schon seit ungefähr dreißig Jahren als Lichtschutzmittel und Stabilisatoren, insbesondere für Kunststoffe und Lacke, kommerziell im Einsatz.

In der WO 94/12544 sind mit HALS-Gruppen substituierte Maleinsäureimid-α-Olefin-Copolymerisate beschrieben, die sich neben einer fehlenden Tendenz zur Migration in dem zu schützenden Material auch durch eine gute Verträglichkeit mit den üblichen Kunststoffarten und durch eine gute Löslichkeit und ausgezeichnete Verträglichkeit in den üblichen Lacksystemen auszeichnen.

Obwohl sich diese Verbindungen in der kommerziellen Praxis bereits hervorragend bewähren, bleibt doch Raum für Verbesserungen, insbesondere was die Lagerungs- und Transporteigenschaften betrifft.

Aufgabe ist daher die Bereitstellung weiterhin verbesserter Lichtschutzmittel, insbesondere mit verbesserten Lagerungs- und Transporteigenschaften.

Die Aufgabe wird gelöst durch eine Mischung der oben beschriebenen oligomeren HALS mit bestimmten niedermolekularen (M < 1000 g/mol) HALS. Die erfindungsgemäße Mischung weist zum einen eine erhöhte, synergistische Stabilisatorwirkung auf, zum anderen zeigt sie verbessert Lagerungs- und Transporteigenschaften, da sie bei der Lagerung, im Gegensatz zu den oligomeren Verbindungen allein, nicht zum Verkleben neigt. Die erfindungsgemäße Mischung braucht daher nicht mit Verdünnungsmitteln, wie pyrogener Kieselsäure, Silikaten, Erdalkalistearaten oder Talk, gestreckt zu werden.

Mischungen von oligomeren und niedermolekularen HALS sind bereits bekannt, zum Beispiel als Tinuvin^{®} 791 der Ciba Specialty Chemicals, dabei handelt es sich jedoch um strukturell unterschiedliche oligomere Verbindungen, die kleinen Rückschluss auf eine vorteilhafte Wirkung der erfindungsgemäßen Mischung zulassen.

Gegenstand der Erfindung ist daher eine Mischung, enthaltend
A. mindestens eine oligomere Verbindung, enthaltend Wiederholeinheiten der Formel (I), 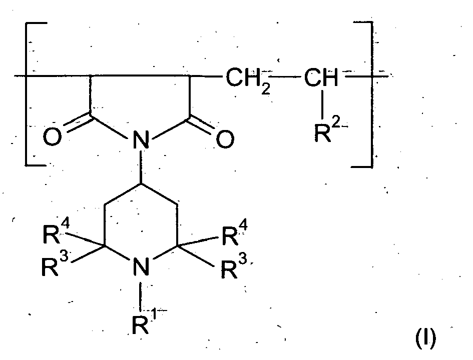 worin die Symbole folgende Bedeutungen haben,
   - R¹: ist Wasserstoff, C₁- bis C₆-Alkyl, Formyl, C₂- bis C₆-Alkanoyl, C₁- bis C₁₂- Alkoxy, C₅- bis C₆-Cycloalkoxy, Cyanomethyl, 2-Hydroxyethyl, Benzyl oder- ein Rest der Formel -CR'=CH-CO-OR", wobei
   - R': Wasserstoff, C₁- bis C₆-Alkyl oder einen Rest der Formel -CO-OR" bedeu- tet und
   - R": C₁- bis C₁₈-Alkyl, C₅- bis C₈-Cycloalkyl, C₇- bis C₁₈-Aralkyl, Phenyl oder To- lyl bedeutet;
   - R²: ist eine Mischung, aus C₁₄- bis C₂₈-Alkylgruppen, wobei zwei dieser Al- kylgruppen, die sich um nicht mehr als zwei C-Atome unterscheiden dürfen, jeweils mindestens 30 % dieser Mischung ausmachen;
   - R³, R⁴: sind unabhängig voneinander C₁- bis C₆-Alkyl;
   und
B. mindestens eine Verbindung der Formel (II) oder (III), 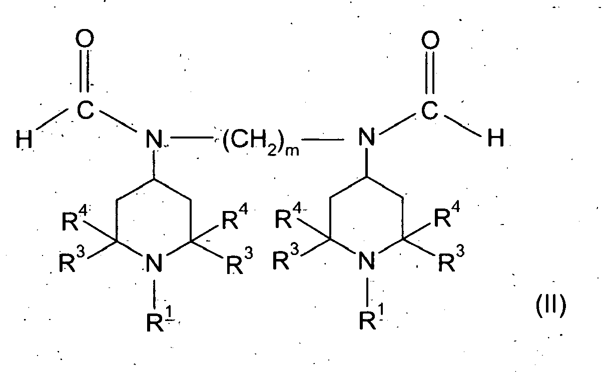 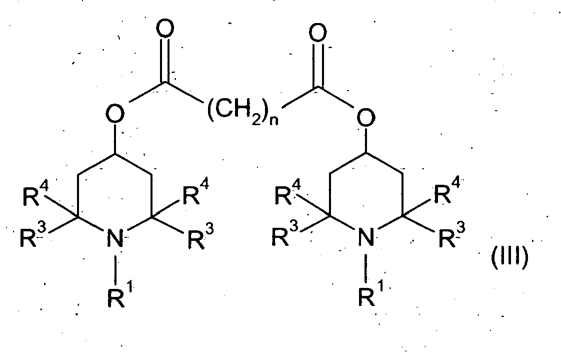
wobei die Symbole und Indizes folgende Bedeutungen haben:
- n, m: sind unabhängig voneinander eine natürliche Zahl von 2 bis 22 und
- R¹, R³, R⁴: haben unabhängig voneinander die in Formel (I) angegebenen Be- deutungen.

Die erfindungsgemäßen Mischungen werden zur Stabilisierung von organischem Material, insbesondere von Polymeren, gegen den Einfluss von Licht eingesetzt.

Die Oligomere (A) weisen im Allgemeinen ein mittleres Molekulargewicht von 1000 bis 50000, bevorzugt von 1500 bis 10000, insbesondere von 2000 bis 5000 auf. Bei den Molekulargewichtsangaben handelt es sich um zahlengemittelte mittlere Molekularewichte.

Die durchschnittliche Zahl der Wiederholeinheiten beträgt 3 bis 100, bevorzugt 4 bis 30, insbesondere 5 bis 10.

Der Rest R¹ ist bevorzugt H, C₁-C₆-Alkyl, Formyl, Acyl, C₁-C₆-Alkoxy oder Benzyl, besonders bevorzugt H, Methyl, Formyl, Acyl oder Benzyl, insbesondere H.

Der Rest R² stellt eine Mischung aus C₁₄- bis C₂₈-Alkylgruppen, vorzugsweise C₁₆- bis C₂₄-Alkylgruppen, insbesondere C₁₈- bis C₂₂-Alkylgruppen dar, dem Copolymerisat liegen also C₁₆- bis C₃₀-α-Olefine, vorzugsweise C₁₈- bis C₂₆-α-Olefine, insbesondere C₂₀-bis C₂₄-α-Olefine als Bausteine zugrunde. Bei R² handelt es sich vorzugsweise um lineare Alkylgruppen.

Das Vorliegen einer Mischung von Alkylgruppen für R² ist so zu verstehen, däss im statistischen Mittel über die Gesamtzahl aller vorliegenden Copolymerisat-Moleküle zwei bestimmte Alkylgruppen, die sich um nicht mehr als zwei C-Atome unterscheiden dürfen, jeweils mindestens 30 %, vorzugsweise jeweils mindestens 40 % dieser Mischung ausmachen. Insbesondere sind dies Mischungen von 3 bestimmten Alkylgruppen, zum Beispiel Octadecyl, Eicosyl und Docosyl, wobei zwei dieser Gruppen, die sich um 2 C-Atome unterscheiden, mehr als 40 % und die dritte Gruppe 3 bis 18% der Mischung ausmachen; hierbei können weitere Alkylgruppen mit etwas weniger als 18 oder etwas mehr als 22 C-Atomen in geringfügigen Mengen, üblicherweise weniger als 2 %, in der Mischung vorliegen.

Falls nichts anderes angegeben wird, handelt es sich bei Prozentangaben um Gewichtsprozente.

Die Reste R³ und R⁴ sind bevorzugt C₁-C₄-Alkyl, besonders bevorzugt Methyl, insbesondere haben jeweils alle Reste R³ und R⁴ die Bedeutung Methyl.

Als Alkylreste, welche in Form von C₁- bis C₆ (für R³ und R⁴) und C₁- bis C₁₈ (für R") angesprochen werden, kommen verzweigte und insbesondere geradkettige Vertreter in Betracht, so vor allem Methyl, Ethyl, n-Propyl, iso-Propyl, n-Butyl, iso-Butyl, sec.-Butyl, tert.-Butyl, n-Amyl, iso-Amyl, sec.-Amyl, tert.-Amyl, Neopentyl, n-Hexyl, n-Heptyl, n-Octyl, 2-Ethylhexyl, 2-Propylheptyl, n-Nonyl, iso-Nonyl, n-Decyl, n-Undecyl, n-Dodecyl, n-Tridecyl, iso-Tridecyl, n-Tetradecyl, n-Pentadecyl, n-Hexadecyl- n-Heptadecyl und n-Octadecyl.

Als geradkettiges oder verzweigtes C₂- bis C₆-Alkanoyl für R¹ kommen vor allem Acetyl, daneben aber auch Propionyl, Butyryl, iso-Butyryl, Pentanoyl und Hexanoyl in Betracht.

Als geradkettige oder verzweigte C₁- bis C₁₂- Alkoxygruppen für R¹ eignen sich vor allem C₆- bis C₈-Alkoxygruppen wie n-Hexoxy, iso-Hexoxy, n-Octoxy, 2-Ethylhexoxy und iso-Octoxy, daneben aber auch Methoxy, Ethoxy, n-Propoxy, iso-Propoxy, n-Butoxy, iso-Butoxy, sec.-Butoxy, tert.-Butoxy, n-Pentoxy, n-Nonoxy, n-Decoxy, n-Undecoxy und n-Dodecoxy.

C₅- bis C₆-Cycloalkoxygruppen für R¹ sind vor allem Cyclopentoxy und Cyclohexoxy.

Als C₅- bis C₈-Cycloalkylreste für R" kommen vor allem Cyclopentyl und Cyclohexyl, daneben auch Cycloheptyl, Cyclooctyl, Methylcyclopentyl, Dimethylcyclopentyl, Methylcyclohexyl, Ethylcyclohexyl und Dimethylcyclohexyl in Betracht.

Als C₇- bis C₁₈-Aralkylreste für R" eignen sich beispielsweise Naphthylmethyl, Diphenylmethyl oder Methylbenzyl, insbesondere jedoch C₇- bis C₁₈-Phehylalkyl wie 1-Phenylethyl, 2-Phenylethyl, 1-Phenylpropyl, 2-Phenylpropyl, 3-Phenylpropyl, 2-Phenyl-prop-2-yl, 4-Phenylbutyl, 2,2-Dimethyl-2-phenylethyl, 5-Phenylamyl, 10-Phenyldecyl, 12-Phenyldodecyl und vorzugsweise Benzyl.

Als Tolylreste kommen ortho-, meta und bevorzugt p-Tolyl in Betracht.

Insbesondere bevorzugt als oligomere Komponente A ist ein Oligomer mit der Wiederholeinheit (Ia) 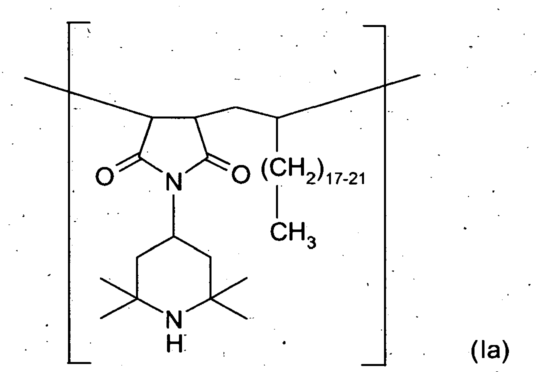 und einer zahlengemittelten Molmasse von 3000 - 4000 g/mol.

Eine solche Verbindung ist unter der Bezeichnung Uvinul^{®} 5050 H von der BASF Aktiengesellschaft, Ludwigshafen, Deutschland erhältlich.

Die Herstellung der oligomeren Komponente (A) kann, falls die Verbindungen nicht kommerziell erhältlich sind, nach den in der WO 94/12544 angegebenen Methoden erfolgen.

Von den niedermolekularen Verbindungen (II) und (III) der Komponente (B) sind solche bevorzugt, bei denen die Symbole und Indizes folgende Bedeutungen haben:
- R¹: ist bevorzugt H, C₁-C₄-Alkyl, Formyl, Acyl oder Benzyl, besonders bevorzugt H, Methyl, Formyl, Acyl oder Benzyl, ganz besonders be- vorzugt H.
- R³ und R⁴: sind bevorzugt C₁-C₄-Alkyl, besonders bevorzugt Methyl, ganz be- sonders bevorzugt R³ und R⁴ Methyl.
- m, n: sind bevorzugt eine natürliche Zahl von 4 bis 10, besonders bevor- zugt 6 oder 8, ganz besonders bevorzugt steht m für die Zahl 6 und n für die Zahl 8.

Bevorzugt sind Verbindungen (II) und (III), bei denen die Symbole und Indizes die bevorzugten Bedeutungen haben. Besonders bevorzugt sind Verbindungen (II) und (III), bei denen die Symbole und Indizes die besonders bevorzugten Bedeutungen haben Ganz besonders bevorzugt sind die Verbindungen, bei denen die Symbole und Indizes die ganz besonders bevorzugten Bedeutungen haben.

Insbesondere bevorzugt als Verbindungen der Komponente (B) sind die Verbindungen (IIa), (IIIa) und (IIIb). 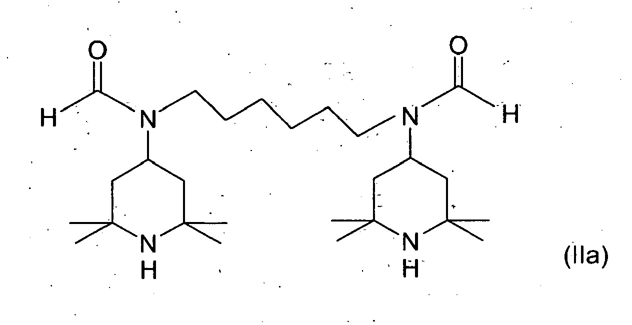 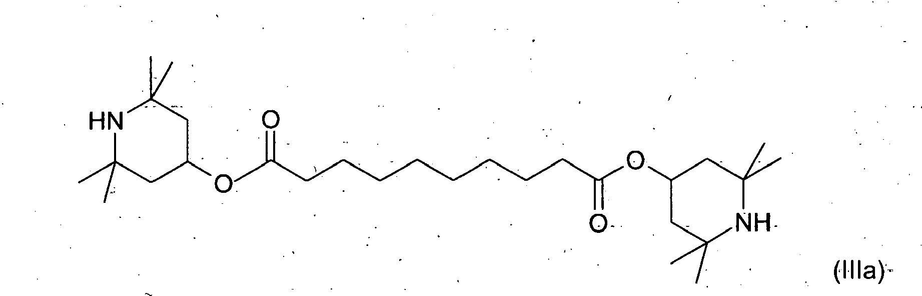 und 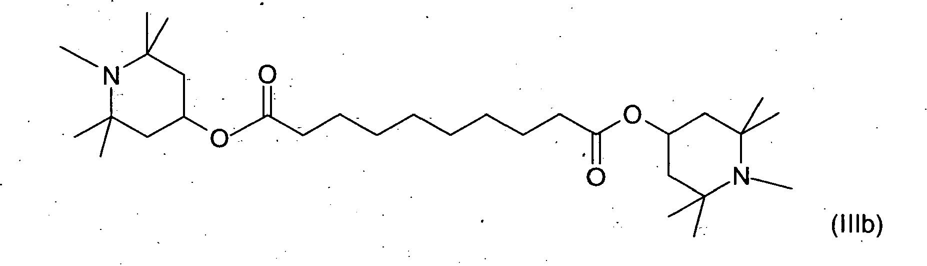 wie sie unter der Bezeichnung Uvinul^{®} 4050 H und Uvinul^{®} 4077 H von der BASF Aktiengesellschaft, Ludwigshafen, Deutschland kommerziell erhältlich sind.

Insbesondere bevorzugt als Komponente B ist die Verbindung (IIa).

Die Herstellung von Verbindungen der Formel (II) kann, soweit sie nicht kommerziell erhältlich sind, nach den in der EP-A 0 316 582 beschriebenen Methoden erfolgen.

Die Herstellung von Verbindungen der Formel (III) kann, soweit sie nicht kommerziell erhältlich sind, nach bekannten, dem Fachmann geläufigen Methoden erfolgen, beispielsweise durch Veresterung von Sebacinsäureestern mit 2,2,6,6-Tetraalkylpiperidin-4-ol-Derivaten.

Das Gewichtsverhältnis der Komponenten (A) und (B) in den erfindungsgemäßen Mischungen beträgt im Allgemeinen 5:1 bis 1:5, bevorzugt 2:1 bis 1:2, besonders bevorzugt 1,2:1 bis 1:1,2, insbesondere bevorzugt ist eine Mischung im Gewichtsverhältnis von etwa 1:1.

Die Herstellung der erfindungsgemäßen Mischungen kann nach bekannten, dem Fachmann geläufigen Verfahren erfolgen.

Vorzugsweise kann man die Komponente (B) einer Schmelze der Komponente (A) zusetzen, homogenisieren, in die gewünschte Form, beispielsweise Pastillen, bringen und erkalten lassen.

Gegenstand der Erfindung ist daher auch ein entsprechendes Verfahren zur Herstellung der erfindungsgemäßen Mischungen.

Man kann aber auch Lösungen der beiden Komponenten (A) und (B) mischen und das oder die Lösungsmittel dann entfernen.

Besonders bevorzugt sind die folgenden Kombinationen von Verbindungen der Komponenten (A) und (B):
a) die Kombination der Verbindungen (la) und (IIa),
b) die Kombination der Verbindungen (la) und (IIIa),
c) die Kombination der Verbindungen (la) und (IIIb).

Insbesondere bevorzugt ist die Kombination (a).

Die erfindungsgemäßen Mischungen eignen sich in hervorragender Weise zur Verwendung als Stabilisatoren zum Stabilisieren von organischem Material (bevorzugt nicht von lebendem organischem Material) gegen die Einwirkung von Licht, Sauerstoff und Wärme. Sie werden den zu stabilisierenden organischen Materialien in einer Konzentration von 0,01 bis 5 Gew.-%, vorzugsweise von 0,02 bis 1 Gew.-%, bezogen auf das organische Material, vor, während oder nach lhrer Herstellung zugesetzt.

Die erfindungsgemäße Mischung kann dem zu schützenden organischen Material als vorgefertigte Mischung der Komponenten (A) und (B) zugesetzt werden, ebenso ist jedoch die getrennte Zugabe der Komponenten (A) und (B) zu dem zu schützenden Material möglich, die Mischung entsteht dann erst in dem zu schützenden Material. Bei einer getrennten Zugabe der Komponenten (A) und (B) kann diese gleichzeitig oder zeitlich versetzt erfolgen, wobei die Reihenfolge im Allgemeinen unerheblich ist.

Unter organischem Material sind beispielsweise kosmetische Präparate wie Salben und Lotionen, Arzneimittelformulierungen wie Pillen und Zäpfchen, photografisches Aufzeichnungsmaterial wie photografische Emulsionen oder Vorprodukte für Kunststoffe und Lacke, insbesondere jedoch Kunststoffe und Lacke selbst, zu verstehen.

Gegenstand der Erfindung ist außerdem ein gegen die Einwirkung von Licht, Sauerstoff und Wärme stabilisiertes organisches Material, insbesondere ein Kunststoff oder Lack, welches eine erfindungsgemäße Mischung, vorzugsweise in den oben angegebenen Konzentrationen, enthält.

Zur Vermischung der erfindungsgemäßen Mischung, insbesondere mit Kunststoffen, können alle bekannten Vorrichtungen und Methoden zum Einmischen von Stabilisierungsmitteln oder anderen Zusätzen in Polymere angewandt werden.

Wahlweise enthält die erfindungsgemäße Mischung oder das durch die Mischung zu stabilisierende organische Material zusätzlich wenigstens einen weiteren Lichtstabilisator und/oder weitere (Co)stabilisatoren. Geeignete Lichtstabilisatoren und weitere (Co)stabilisatoren sind beispielsweise aus den Gruppen a) bis s) ausgewählt:
a) 4,4-Diarylbutadiene,
b) Zimtsäureester,
c) Benzotriazole,
d) Hydroxybenzophenone,
e) Diphenylcyanacrylate,
f) Oxamide,
g) 2-Phenyl-1,3,5-triazine;
h) Antioxidantien,
i) Nickelverbindungen
j) weitere sterisch gehinderte Amine,
k) Metalldesaktivatoren,
l) Phosphite und Phosphonite,
m) Hydroxylamine,
n) Nitrone,
o) Aminoxide,
p) Benzofuranone und Indolinone,
q) Thiosynergisten,
r) Peroxid-zerstörende Verbindungen und
s) basische Costabilisatoren.

Zur Gruppe a) der 4,4-Diarylbutadiene zählen beispielsweise Verbindungen der Formel (aa)

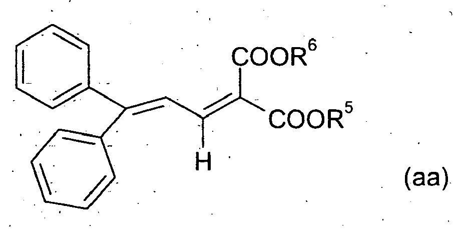

Die Verbindungen sind aus der EP-A-916 335 bekannt. Die Substituenten R⁵ und/oder R⁶ bedeuten bevorzugt C₁-C₈-Alkyl und C₅-C₈-Cycloalkyl.

Zur Gruppe b) der Zimtsäureester zählen beispielsweise 4-Methoxyzimtsäure-2-isoamylester, 4-Methoxyzimtsäure-2-ethylhexylester, Methyl-α-methoxycarbonylcinnamat, Methyl-α-cyano-β-methyl-p-methoxycinnamat, Butyl-α-cyano-β-methyl-p-methoxycinnamat und Methyl-α-methoxycarbonyl-p-methoxycinnamat.

Zur Gruppe c) des Benzotriazole zählen beispielsweise 2-(2'-Hydroxyphenyl)-benzotriazole wie 2-(2'-Hydroxy-5'-methylphenyl)-benzotriazol, 2-(3',5'-Di-tert-butyl-2'-hydroxyphenyl)benzotriazol, 2-(5'-tert-Butyl-2'-hydroxyphenyl)-benzotriazol, 2-(2'-Hydroxy-5'-(1,1,3,3-tetramethylbutyl)phenyl)benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenylybenzotriazol, 2-(2'-Hydroxy-4'-octyloxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, 2-(3'-tert-Butyl-2'-hydroxy-5'-(2-octyloxycarbonylethyl)phenyly)-5-chlorbenzotriazol, 2-(3'-tert-Butyl-5'-[2-(2-ethylhexyloxy)-carbonylethyl]-2'-hydroxyphenyl)-5-chlorbenzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol und 2-(3'-tert-Butyl-2'-hydroxy-5'-(2-isooctyloxycarbonylethyl)-phenylbenzotriazol, 2,2'-Methylen-bis[4-(1,1,3,3-tetramethylbutyl)-6-benzotriazol-2-yl-phenol]; das Produkt der Veresterung von 2-[3'-tert-Butyl-5'-(2-methoxycarbonylethyl)-2'-hydroxyphenyl]-2H-benzotriazol mit Polyethylenglycol 300; [R-CH₂CH₂-COO(CH₂)₃]₂, mit R = 3'-tert-Butyl-4'-hydroxy-5'-2H-benzotriazol-2-yl-phenyl und Gemische davon.

Zur Gruppe d) der Hydroxybenzophenone zählen beispielsweise 2-Hydroxybenzophenone wie 2-Hydroxy-4-methoxybenzophenon, 2,2'-Dihydroxy-4-methoxybenzophenon, 2,4-Dihydroxybenzophenon, 2,2',4,4'-Tetrahydroxybenzophenon, 2,2'-Dihydroxy-4,4'-dimethoxybenzophenon, 2,2'-Dihydroxy-4,4'-dimethoxybenzophenon, 2-Hydroxy-4-(2-ethylhexyloxy)benzophenon, 2-Hydroxy-4-(n-octyloxy)benzophenon, 2-Hydroxy-4-methoxy-4'-methylbenzophenon, 2-Hydroxy-3-carboxybenzophenon, 2-Hydroxy-4-methoxybenzophenon-5-sulfonsäure und deren Natriumsalz, 2,2'-Dihydroxy-4,4'-dimethoxybenzophenon-5,5'-bissulfonsäure und deren Natriumsalz.

Zur Gruppe e) der Diphenylcyanacrylate zählen beispielsweise Ethyl-2-cyan-3,3-diphenylacrylat, das beispielsweise im Handel unter dem Namen Uvinul® 3035 der Fa. BASF Aktiengesellschaft, Ludwigshafen erhältlich ist, 2-Ethylhexyl-2-cyan-3,3-diphenylacrylat, das beispielsweise im Handel als Uvinul® 3039 der Fa. BASF Aktiengesellschaft, Ludwigshafen, erhältlich ist und 1,3-Bis-[(2'-cyano-3',3'-diphenylacryloyl)oxy]-2,2-bis{[2'-cyano-3',3'-diphenylacryloyl)oxy]methyl}propan, das beispielsweise im Handel unter dem Namen Uvinul® 3030 der Fa. BASF Aktiengesellschaft, Ludwigshafen erhältlich ist.

Zur Gruppe f) der Oxamide zählen beispielsweise 4,4'-Dioctyloxyoxanilid, 2,2'-Diethoxyoxanilid, 2,2'-Dioctyloxy-5,5'-di-tert-butoxanilid, 2,2'-Didodecyloxy-5,5'-di-tert-butoxanilid, 2-Ethoxy-2'-ethyloxanilid, N,N'-Bis(3-dimethylaminopropyl)oxamid, 2-Ethoxy-5-tert-butyl-2'-ethoxanilid und dessen Mischung mit 2-Ethoxy-2'-ethyl-5,4'-ditert-butoxanilid sowie Mischungen von ortho-, para-Methoxy-disubstituierten Oxaniliden und Mischungen von ortho- und para-Ethoxy disubstituierten Oxaniliden.

Zur Gruppe g) der 2-Phenyl-1,3,5-triazine zählen beispielsweise 2-(2-Hydroxyphenyl)-1,3,5-triazine wie 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propoxy)-phenyl]-4,6-bis(2,4-dimethylphenyl)-1,3,5-triazin, 2-[2-Hydroxy-4-(2-hydroxy-3-octyloxy-propoxy)phenyl]-4,6-bis(2,4-dimethylphenyl)-1,3,5-triazin, 2-[4-(Dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis-(2,4-dimethylphenyl)-1,3,5-triazin, 2-(2-Hydroxy-4-hexyloxyphenyl)-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin und 2-(2-Hydroxyphenyl)-4-(4-methoxyphenyl)-6-phenyl-1,3,5-triazin.

Die Gruppe h) der Antioxidantien umfasst beispielsweise:
h.1) Alkylierte Monophenole wie beispielsweise 2,6-Di-tert-butyl-4-methylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-tert-butyl-4-methoxymethylphenol, unverzweigte oder in der Seitenkette verzweigte Nonylphenole wie beispielsweise 2,6-Di-nonyl-4-methylphenol, 2,4-Dimethyl-6-(1-methylundec-1-yl)-phenol, 2,4-Dimethyl-6-(1-methylheptadec-1-yl)-phenol, 2,4-Dimethyl-6-(1-methyltridec-1-yl-)phenol und Gemische davon.
h.2) Alkylthiomethylphenole wie zum Beispiel 2,4-Dioctylthiomethyl-6-tert-butylphenol, 2;4-Dioctylthiomethyl-6-methylphenol, 2,4-Dioctylthiomethyl-6-ethylphenol, 2;6-Didodecylthiomethyl-4-nonylphenol.
h.3) Hydrochinone und alkylierte Hydrochinone wie zum Beispiel 2,6-Di-tert-butyl-4-methoxyphenol, 2,5-Di-tert-butylhydrochinon, 2,5-Di-tert-amylhydrochinon, 2,6-Diphenyl-4-octadecyloxyphenol, 2,6-Di-tert-butylhydrochinon, 2,5-Di-tert-butyl-4-hydroxyanisol, 3,5-Di-tert-butyl-4-hydroxyanisol, 3,5-Di-tert-butyl-4-hydroxyphenylstearat, Bis-(3,5-di-tert-butyl-4-hydroxyphenyl)adipat.
h.4) Tocopherole, wie zum Beispiel α-Tocopherol, β-Tocopherol, γ-Tocopherol, δ-Tocopherol und Gemische davon (Vitamin E).
h.5) Hydroxylierte Thiodiphenylether wie zum Beispiel 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis(6-tert-butyl-2-methylphenol), 4,4'-Thio-bis-(3,6-disec-amylphenol), 4,4'-Bis(2,6-dimethyl-4-hydroxyphenyl)disulfid.
h.6) Alkyliden-Bisphenole wie zum Beispiel 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)butan, 1,1-Bis(5-tert-butyl-4-hydroxy-2-methyl-phenyl)-3-n-dodecylmercaptobutan, Ethylenglycol-bis-[3,3-bis(3-tert-butyl-4-hydroxyphenyl)butyrat], Bis(3-tert-butyl-4-hydroxy-5-methyl-phenyl)dicyclopentadien, Bis[2-(3'-tert-butyl-2-hydroxy-5- methylbenzyl)-6-tert-butyl-4-methylphenyl]terephthalat, 1,1-bis-(3, 5-dimethyl-2-hydroxyphenyl)butan, 2,2-Bis-(3,5-di-tert-butyl-4-hydroxyphenyl)propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
h.7) Benzylverbindungen wie zum Beispiel 3,5,3',5'-Tetra-tert-butyl-4,4'-dihydroxydibenzylether, Octadecyl-4-hydroxy-3,5-dimethylbenzylmercaptoacetat, Tridecyl-4-hydroxy-3,5-di-tert-butylbenzylmercaptoacetat, Tris(3,5-di-tert-butyl-4-hydroxybenzyl)amin, 1,3,5-Tri-(3,5-di-tert-butyl-4-hydroxybenzyl)-2,4,6-trimethylbenzol, Di-(3,5-Di-tert-butyl-4-hydroxybenzyl)sulfid, 3,5-Di-tert-butyl-4-hydroxybenzyl-mercapto-essigsäureisooctylester, Bis-(4-tert-butyl-3-hydroxy-2,6-dimethylbenzyl)dithiolterephthalat, 1,3,5-Tris-(3,5-di-tert-butyl-4-hydroxybenzyl)isocyanurat, 1,3,5-Tris-(4-tert-butyl-3-hydroxy-2, 6-dimethylbenzyl)isocyanurat, 3,5-Di-tert-butyl-4-hydroxybenzyl-phosphorsäuredioctadecyl-ester und 3,5-Di-tert-butyl-4-hydroxybenzyl-phosphorsäuremonoethylester, Calciumsalz.
h.8) Hydroxybenzylierte Malönate wie zum Beispiel Dioctadecyl-2,2-bis-(3,5-di-tert butyl-2-hydroxybenzylymalonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)malonat, Bis[4-(1,1,3,3-tetramethylbutyl)phenyl]-2,2-bis(3,5-di-tert-butyl-4-hydroxybenzyl)malonat.
h.9) Hydroxybenzyl-Aromaten wie zum Beispiel 1,3,5-Tris-(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)phenol.
h.10)Triazinverbindungen wie zum Beispiel 2,4-Bis(octylmercapto)-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxyl)-1,3,5-triazin, 1,3,5-Tris-(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.
h.11)Benzylphosphonate wie zum Beispiel Dimethyl-2,5-di-tert-butyl-4-hydroxybenzylphosphonat, Diethyl-3,5-di-tert-butyl-4-hydroxybenzylphosphonat ((3,5-Bis(1,1-dimethylethyl)-4-hydroxyphenyl)methyl)Iphosphonsäurediethylester), Dioctadecyl-3,5-di-tert-butyl-4-hydroxybenzylphosphonat, Dioctadecyl-5-tert-butyl-4-hydroxy-3-methylbenzylphosphonat, Calciumsalz des 3,5-Di-tert-butyl-4-hydroxybenzylphosphonsäure-monoethylesters.
h.12)Acylaminophenole wie zum Beispiel 4-Hydroxy-laurinsäureanilid, 4-Hydroxystearinsäureanilid, 2,4-Bis-octylmercapto-6-(3,5-tert-butyl-4-hydroxyanilino)-s-triazin und Octyl-N-(3,5-di-tert-butyl-4-hydroxyphenyl)-carbamat.
h.13)Ester der β-(3,5-Di-tert-butyl-4-hydroxyphenyl)propionsäure mit ein- oder mehrwertigen Alkoholen wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(Hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.
h.14)Ester der β-(5-tert-Butyl-4-hydroxy-3-methylphenyl)propionsäure mit ein- oder mehrwertigen Alkoholen wie z. B. mit Methanol, Ethanol, n-Octanol, i-Octanol, Octadecanol, 1,6-Hexaridiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.
h.15)Ester der β-(3,5-Dicyclohexyl-4-hydroxyphenyl)propionsäure mit ein- oder mehrwertigen Alkoholen wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo [2.2.2]octan.
h.16)Ester der 3,5-Di-tert-butyl-4-hydroxyphenylessigsäure mit ein- oder mehrwertigen Alkoholen wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexanediol, Trimethylolpropan, 4-Hydroxymethyl-1-phospha-2,6,7-trioxabicyclo[2.2.2]octan.
h.17)Amide der β-(3, 5-Di-tert-butyl-4-hydroxyphenyl)propionsäure wie z. B. N,N'-Bis(3,5-di-tert-butyl-4-hydroxyphenylpropionyl)-hexamethylendiamin, N,N'-Bis(3,5-di-tert-butyl-4-hydroxyphenylpropionyl)-trimethylendiamin, N,N'-Bis(3,5-di-tert-butyl-4-hydroxyphenylpropionyl)-hydrazin, N,N'-Bis[2-(3-[3,5-di-tert-butyl-4-hydroxyphenyl]-propionyloxy)ethyl]-oxamid (z. B. Naugard®XL-1 der Firma Uniroyal).
h.18)Ascorbinsäure(Vitamin C)
h.19)Aminische Antioxidantien wie zum Beispiel N,N'-Di-isopropyl-p-phenylendiamin, N,N'-Di-sec-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N, N'-Dicyclohexyl-p-phenylendiamin, N, N'-Diphenyl-p-phenylendiamin, N,N,'-Bis(2-naphthyl)-p-phenylendiamin, N-Isopropyl-N'-phenyl-p-phenylendiamin, N-(1,3-Dimethylbutyl)-N'-phenyl-p-phenylendiamin, N-(1-Methylheptyl)-N'-phenyl-p-phenylendiamin, N-Cyclohexyl-N'-phenyl-p-phenylendiamin, 4-(p-Toluolsulfamoyl)diphenylamin, N,N'-Dimethyl-N,N'-di-sec-butyl-p-phenylendiamin, Diphenylamin, N-Allyldiphenylamin, 4-Isopropoxydiphenylamin, N-Phenyl-1-naphthylamin, N-(4-tert-Octylphenyl)-1-naphthylamin, N-Phenyl-2-naphthylamin, octyliertes Diphenylamin, zum Beispiel p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylaminophenol, Bis-(4-methoxyphenyl)amin, 2,6-Di-tert-butyl-4-dimethylaminomethylphenol, 2,4'-Diaminodiphenylmethan, 4,4'-Diaminodiphenylmethan, N,N,N',N'-Tetramethyl-4,4'-diaminodiphenylmethan, 1,2-Bis-[(2-methylphenyl)amino]ethan,1,2-Bis(phenylamino)-propan, (o-Tolyl)-biguanid, Bis[4-(1',3'-dimethylbutyl)phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemisch aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octylphenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, 2,2,6,6-Tetramethylpiperidin-4-on, 2,2,6,6 Tetramethylpiperidin-4-ol, das Dimethylsuccinat-Polymer mit 4-Hydroxy-2,2,6,6-tetramethyl-1-piperidinethanol [CAS Nummer 65447-77-0], (beispielsweise Tinuvin® 622 der Fa. Ciba Specialty Chemicals, Inc.), Polymer of 2,2,4,4-Tetramethyl-7-oxa-3,20-diaza-dispiro[5.1.11.2]-heeicosan-21-on und Epichlorhydrin [CAS-No.: 202483-55-4], beispielsweise (Hostavin® N 30 der Fa. Clariant).

Zur Gruppe i) der Nickelverbindungen gehören zum Beispiel Nickel-Komplexe des 2,2'-Thio-bis-[4-(1,1,3,3-tetramethylbutyl)phenols], wie der 1:1 oder 1:2 Komplex, gegebenenfalls mit zusätzlichen Liganden wie n-Butylamin, Triethanolamin oder N-Cyclohexyldiethanolamin, Nickeldibutyldithiocarbamat, Nickelsalze von 4-Hydroxy-3,5-di-tert-butylbenzylphosphonsäuremonoalkylester wie z. B. der Methyl- oder Ethylester, Nickelkomplexe von Ketoximen wie z. B. von 2-Hydroxy-4-methylphenylundecylketoxim, Nickelkomplex von 1-Phenyl-4-lauroyl-5-hydroxypyrazol, gegebenenfalls mit zusätzlichen Liganden.

Zur Gruppe j) der sterisch gehinderten Amine gehören zum Beispiel 4-Hydroxy-2,2,6,6-tetramethylpiperidin, 1-Allyl-4-hydroxy-2,2,6,6-tetramethylpiperidin, 1-Benzyl-4-hydroxy-2,2,6,6-tetramethylpiperidin, Bis(2,2,6,6-tetramethyl-4-piperidyl)sebacat, Bis(2,2,6,6-tetramethyl-4-piperidyl)succinat, Bis(1,2,2,6,6-pentamethyl-4-piperidyl)sebacat, Bis(1-octyloxy-2, 2,6,6-tetramethyl-4-piperidyl)sebacat, Bis(1,2,2,6,6-pentamethyl-4-piperidyl)-n-butyl-3,5-di-tert-butyl-4-hydroxybenzylmalonat (n-Butyl-3,5-di-tert-butyl-4-hydroxy-benzyl-malonsäure-bis(1,2,2,6,6-pentamethylpiperidyl)-ester), Kondensationsprodukt aus 1-(2-Hydroxyethyl)-2,2,6,6-tetramethyl-4-hydroxypiperidin und Bernsteinsäure, lineare oder cyclische Kondensationsprodukte aus N,N'-Bis(2,2,6,6-tetramethyl-4-piperidyl)hexamethylendiamin und 4-tert-Octylamino-2,6-dichlor-1,3,5-triazin, Tris(2,2,6,6-tetramethyl-4-piperidyl)-nitrilotriacetat, Tetrakis(2,2,6,6-tetramethyl-4-piperidyl)-1,2,3,4-butan-tetracarboxylat, 1,1'-(1, 2-Ethandiyl)-bis(3,3,5,5-tetramethylpiperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert butylbenzyl)malonat, 3-n-Octyl-7,7,9,9-tetramethyl-1,3,8-triazaspiro [4.5]decan-2,4-dion, Bis-(1-octyloxy-2,2,6,6-tetramethylpiperidyl)-sebacat, Bis-(1-octyloxy-2,2,6,6-tetramethyl-piperidyl)-succinat, lineare oder cyclische Kondensationsprodukte von N,N'-Bis-(2,2,6,6-tetramethyl-4-piperidyl)hexamethylendiamin und 4-Morpholino-2,6-dichlor-1,3,5-triazin, Kondensationsprodukt von N,N'-Bis-(2,2,6,6-tetramethyl-4-piperidyl)hexamethylendiamin und Ameisensäureester (CAS-Nr. 124172-53-8, z.B. Uvinul® 4050H der Fa. BASF Aktiengesellschaft, Ludwigshafen), Kondensationsprodukt von 2-Chlor-4,6-bis(4-n-butylamino-2, 2,6,6-tetramethylpiperidyl)-1,3,5-triazin und 1,2-Bis(3-aminopropylamino)ethan, Kondensationsprodukt von 2-Chlor-4,6-di-(4-n-butylamino-1,2,2,6,6-pentamethylpiperidyl)-1,3,5-triazin und 1, 2-Bis-(3-amino-propylamino)ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)pyrrolidin-2,5-dion, Gemisch von 4-Hexadecyloxy- und 4-Stearyloxy-2,2,6,6-tetramethylpiperidin, Kondensationsprodukt aus N,N'-Bis(2,2,6,6-tetramethyl-4-piperidyl)hexamethylendiamin und 4-Cyclohexylamino-2,6-dichlor-1,3,5-triazin, Kondensationsprodukt aus 1,2-Bis(3-aminopropylamino)ethan und 2,4,6-Trichlor-1,3,5-triazin sowie 4-Butylamino-2,2,6,6-tetramethylpiperidin (CAS Reg. No. [136504-96-6]); N-(2,2,6,6-Tetramethyl-4-piperidyl)-n-dodecylsuccinimid, N-(1,2,2,6,6-Pentamethyl-4-piperidyl)-n-dodecylsuccinimid, 2-Undecyl-7,7,9,9-tetramethyl-1-oxa-3,8-diaza-4-oxospiro[4,5]decan, Reaktionsprodukt von 7,7,9,9-Tetramethyl-2-cycloundecyl-1-oxa-3,8-diaza-4-oxospiro[4, 5]decan und Epichlorhydrin, 1,1-Bis(1,2,2,6,6-pentamethyl-4-piperidyloxycarbonyl)-2-(4-methoxyphenyl)ethen, Diester der 4-Methoxy-methylenmalonsäure mit 1,2,2,6,6-Pentamethyl-4-hydroxypiperidin, Poly[methylpropyl-3-oxo-4-(2,2,6,6-tetramethyl-4-piperidyl)]siloxan, Reaktionsprodukt aus Maleinsäureanhydrid-α-Olefin-copolymer und 2,2,6,6-Tetramethyl-4-aminopiperidin oder 1,2,2,6,6-Pentamethyl-4-aminopiperidin, 1-(2-Hydroxy-2-methylpropoxy)-4-octadecanoyloxy-2,2,6,6-tetramethylpiperidin, 1-(2-Hydroxy-2-methylpropoxy)-4-hexadecanoyloxy-2, 2,6,6-tetramethylpiperidin, das Reaktionsprodukt aus 1-Oxyl-4-hydroxy-2,2,6,6-tetramethylpiperidin und einem Kohlenstoffrest von t-Amylalkohol, 1-(2-Hydroxy-2-methylpropoxy)-4-hydroxy-2,2,6,6-tetramethylpiperidin, 1-(2-Hydroxy-2-methylpropoxy)-4oxo-2,2,6,6-tetramethylpiperidin, Bis(1-(2-hydroxy-2-methylpropoxy)-2,2,6,6-tetramethylpiperidin-4-yl)sebacat, Bis(1-(2-hydroxy-2-methylpropoxy)-2, 2,6,6-tetramethylpiperidin-4-yl)adipat, Bis(1-(2-hydroxy-2-methylpropoxy)-2, 2,6,6-tetramethylpiperidin-4-yl)succinat, Bis(1-(2-hydroxy-2-methylpropoxy)-2, 2,6,6-tetramethylpiperidin-4-yl)glutarat, 2,4-bis{N[1-(2-hydroxy-2-methylpropoxy)-2,2,6,6-tetramethylpiperidin-4-yl]-N-butylamino}-6-(2hydroxyethyl-amino)-s-triazin, N,N'-Bis-formyl-N,N'-bis(1,2,2,6,6-pentamethyl-4-piperidyl)-hexamethylendiamin, Hexahydro-2,6-bis(2,2,6,6-tetramethyl-4-piperidyl)-1H,4H,5H,8H-2,3a,4a,6,7a,8a-hexaazacyclopenta[def]fluoren-4,8-dion (z. B. Uvinul® 4049 der Fa. BASF Aktiengesellschaft, Ludwigshafen), Poly[[6-[(1,1,3,3-tetramethylbutyl)amino]-1,3,5-triazin-2,4-diyl][(2,2,6,6-tetramethyl-4-piperidinyl)imino]1,6-hexandiyl[(2,2,6,6-tetramethyl-4-piperidinyl)imino]]) [CAS Nr. 71878-19-8], 1,3,5-Triazin-2,4,6-triamin,N,N"'-[1,2-ethan-diyl-bis [[4,6-bis-[butyl(1,2,2,6,6-pentamethyl-4-piperidinyl)amino]-1,3,5-triazin-2-yl]imino]-3,1-propandiyl]]bis[N',N"-dibutyl-N',N"-bis(1,2,2,6,6-pentamethyl-4-piperidinyl)- (CAS Nr. 106990-43-6) (z. B. Chimassorb 119 der Fa. Ciba Specialty Chemicals, Inc.).

Zur Gruppe k) der Metalldesaktivatoren gehören zum Beispiel N,N'-Diphenyloxalsäurediamid, N-Salicylal-N'-salicyloyl-hydrazin, N,N'-Bis(salicyloyl)hydrazin, N, N'-Bis(3,5-di-tert-butyl-4-hydroxyphenylpropionyl)hydrazin, 3-Salicyloylamino-1,2,4-triazol, Bis(benzyliden)-oxalyldihydrazid, Oxanilid, Isophthaloyldihydrazid, Sebacoylbisphenylhydrazid, N, N'-Diacetyladipinsäuredihydrazid, N,N'-Bis(salicyloyl)oxalsäuredihydrazid, N,N'-Bis(salicyloyl)thiopropionyldihydrazid.

Zur Gruppe I) der Phosphite und Phosphonite gehören zum Beispiel Triphenylphosphit, Diphenylalkylphosphite, Phenyldialkylphosphite, Tris(nonylphenyl)phosphit, Trikaurylphosphit, Trioctadecylphosphit, Distearylpentaerythritdiphosphit, Tris(2,4-di-tert-butylphenyl)phosphit, Diisodecylpentaerythritdiphosphit, Bis(2,4-di-tert-butylphenyl)pentaerythritdiphosphit, Bis(2,6-di-tert-butyl-4-methylphenyl)-pentaerythritdiphosphit, Diisodecyloxypentaerythritdiphosphit, Bis(2,4-di-tert-butyl-6-methylphenyl)-pentaerythritdiphosphit, Bis(2,4,6-tris(tert-butylphenyl)pentaerythritdiphosphit, Tristearylsorbittriphosphit, Tetrakis-(2,4-di-tert-butylphenyl)-4,4'-biphenylendiphosphonit, 6-lsooctyloxy-2,4,8,10-tetra-tert-butyl-dibenz[d,f][1,3,2]dioxaphosphepin, 6-Fluor-2,4,8,10-tetra-tert-butyl-12-methyl-dibenz[d,g][1,3,2]dioxaphosphocin, Bis(2,4-di-tert-butyl-6-methylphenyl)methylphosphit, Bis(2,4-di-tert-butyl-6-methylphenyl)ethyl-phosphit, 2,2',2"-Nitrilo[triethyl-tris(3,3', 5,5'-tetra-tert-butyl-1,1'-biphenyl-2,2'-diyl)phosphit], 2- Ethylhexyl-(3,3', 5,5'-tetra-tert-butyl-1,1'-biphenyl-2,2'-diiyl)phosphit.

Zur Gruppe m) der Hydroxylamine gehören zum Beispiel N, N-Dibenzylhydroxylamin, N, N-Diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N-Methyl-N-octadecylhydroxylamin und N, N-Dialkylhydroxylamin aus hydrierten Talgfettaminen.

Zur Gruppe n) der Nitrone gehören zum Beispiel N-Benzyl-α-phenylnitron, N-Ethyl-αmethylnitron, N-Octyl-α-heptylnitron, N-Lauryl-α-undecylnitron, N-Tetradecyl-αtridecylnitron, N-Hexadecyl-α-pentadecylnitron, N-Octadecyl-α-heptadecylnitron, N-Hexadecyl-α-heptadecylnitron, N-Ocatadecyl-α-pentadecylnitron, N-Heptadecyl-αheptadecylnitron, N-Octadecyl-α-hexadecylnitron, N-Methyl-α-heptadecylnitron und Nitrone, abgeleitet von N,N-Dialkylhydroxylaminen hergestellt aus hydrierten Talgfettaminen.

Zur Gruppe o) der Aminoxide gehören zum Beispiel Aminoxidderivate wie sie in den U.S. Patenten Nr. 5,844,029 und 5,880,191 beschrieben sind, Didecylmethylaminoxid, Tridecylaminoxid, Tridodecylaminoxid und Trihexadecylaminoxid.

Zur Gruppe p) der Benzofuranone und Indolinone gehören zum Beispiel'die in den US-Patenten 4,325,863; 4,338,244; 5,175,312; 5,216,052; 5,252,643; in der DE-A 4316611; in der DE-A 4316622; in der DE-A 4316876; in der EP-A 0589839 oder EP-A 0591102 beschriebenen, oder 3-[4-(2-Acetoxyethoxy)phenyl]-5,7-di-tert-butylbenzofuran-2-on, 5,7-Di-tert-butyl-3-[4-(2-stearoyloxyethoxy)phenyl]benzofuran-2-on, 3,3'-Bis[5,7-di-tert-butyl-3-(4-[2-hydroxyethoxy-]phenyl)benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxyphenyl)-5,7-di-tert-butyl-benzofuran-2-on, 3-(3,4-Dimethylphenyl)-5,7-di-tert-butyl-benzofüran-2-on, Irganoxs HP-136 der Firma Ciba Specialty Chemicals, und 3-(2,3-Dimethylphenyl)-5,7-di-tert-butylbenzofuran-2-on.

Zur Gruppe q) der Thiosynergisten gehören zum Beispiel Dilaurylthiodipropionat oder Distearylthiodipropionat.

Zur Gruppe r) der peroxidzerstörende Verbindungen gehören zum Beispiel Ester der β-Thiodipropionsäure, zum Beispiel der Lauryl-, Stearyl-, Myristyl- oder Tridecylester, Mercaptobenzimidazol oder das Zinksalz des 2-Mercaptobenzimidazols, Zinkdibutyldithiocarbamat, Dioctadecyldisulfid, Pentaerythrit-tetrakis(β-dodecylmercaptopropionat).

Zur Gruppe s) der basischen Costabilisatoren gehören zum Beispiel Melamin, Polyvinylpyrrolidon, Dicyandiamid, Triallylcyanurat, Harnstoffderivate, Hydrazinderivative, Amine, Polyamide, Polyurethane, Alkali- und Erdalkalisalze höherer Fettsäuren, zum Beispiel Calciumstearat, Zinkstearat, Magnesiumbehenat, Magnesiumstearat, Natriumricinoleat und Kaliumpalmitat, Antimonbrenzcatechinat oder Zinkbrenzcatechinat.

Weiterhin kann der Kunststoff sonstige Additive und Zusatzstoffe enthalten. Als geeignete Additive der Gruppe t) kommen die üblichen Zusatzstoffe, wie z. B., Pigmente, Farbstoffe, Nukleierungsmittel, Füll- oder Verstärkungsmittel, Beschlagverhinderungsmittel, Biozide und Antistatika, in Betracht.

Geeignete Pigmente sind anorganische Pigmente, beispielsweise Titandioxid in seinen drei Modifikationen Rutil, Anatas oder Brookit, Ultramarinblau, Eisenoxide, Bismutvanadate oder Ruß sowie die Klasse der organischen Pigmente, beispielsweise Verbindungen aus der Klasse der Phthalocyanine, Perylene, Azoverbindungen, Isoindoline, Chinophthalone, Diketopyrrolopyrrole, Chinacridone, Dioxazine, Indanthrone.

Unter Farbstoffen sind alle Farbmittel zu verstehen, die sich im verwendeten Kunststoff vollständig lösen bzw. in einer molekulardispersen Verteilung vorliegen und somit zur hochtransparenten, nichtstreuenden Einfärbung von Polymeren verwendet werden können. Ebenfalls als Farbstoffe sind organische Verbindungen anzusehen, die eine Fluoreszenz im sichtbaren Teil des elektromagnetischen Spektrums aufweisen wie Fluoreszenzfarbstoffe.

Geeignete Nukleierungsmittel umfassen zum Beispiel anorganische Stoffe, beispielsweise Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z. B. 4-tert-Butylbenzoesäure, Adipinsäure, Diphenylessigsäure, Natriumsuccinat oder Natriumbenzoat; polymere Verbindungen, wie beispielsweise ionische Copolymerisate ("Ionomere").

Geeignete Füll- oder Verstärkungsstoffe umfassen zum Beispiel Calciumcarbonat, Silikate, Tälk, Mica, Kaolin, Glimmer, Bariumsulfat, Metalloxide und -hydroxide, Ruß, Graphit, Holzmehl und Mehle oder Fasern anderer Naturprodukte, synthetische Fasern. Als Beispiele für faserförmige bzw. pulverförmige Füllstoffe kommen außerdem Kohlenstoff- oder Glasfasern in Form von Glasgeweben, Glasmatten oder Glasseidenrovings, Schnittglas, Glaskugeln sowie Wollastonit in Betracht. Die Einarbeitung von Glasfasern kann sowohl in Form von Kurzglasfasern als auch in Form von Endlosfasern (Rovings) erfolgen.

Geeignete Antistatika sind beispielsweise Aminderivate wie N,N-Bis(hydroxyalkyl)alkylamine oder -alkylenamine, Polyethylenglycolester und -ether, ethoxylierte Carbonsäureester- und -amide und Glycerinmono- und -distearate sowie deren Mischungen.

Als Kunststoffe, die durch die erfindungsgemäßen Mischungen stabilisiert werden können, seien beispielsweise genannt:
Polymere von Mono- und Diolefinen, wie zum Beispiel Polyethylen niedriger und hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie zum Beispiel Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol sowie Copolymere von Styrol oder α-Methylstyrolmit Dienen und/oder Acrylderivaten, wie zum Beispiel Styrol-Butadien, Styrol-Acrylnitril (SAN), Styrol-Ethylmethacrylat, Styrol-Butadien-Ethylacrylat, Styrol-Acrylnitril-Methacrylat, AcrylnitrilButadien-Styrol (ABS) oder Methylmethacrylat-Butadien-Styrol (MBS);
Halogenhaltige Polymere, wie zum Beispiel Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, zum Beispiel Polyvinylalkohol und Polyvinylacetat;
Polyurethane, Polyamide, Polyharnstoffe, Polyester, Polycarbonate, Polysulfone, Polyethersulfone und Polyetherketone.

Weiterhin können mit den erfindungsgemäßen Mischungen Lacküberzüge stabilisiert werden, zum Beispiel Industrielackierungen. Unter diesen sind Einbrennlackierungen, unter diesen wiederum Fahrzeuglackierungen, vorzugsweise Zweischichtlackierungen, besonders hervorzuheben. Ein anderes Verwendungsgebiet sind zum Beispiel Anstrichmittel für Außenanstriche von Gebäuden, anderen. Bauwerken oder technischen Apparaturen.

Die erfindungsgemäßen Mischungen können in fester oder gelöster Form dem Lack zugesetzt werden. Ihre gute. Löslichkeit in Lacksystemen ist dabei von besonderem Vorteil.

Bevorzugt werden die erfindungsgemäßen Mischungen zum Stabilisieren von Polyamiden sowie ABS- und SAN-Polymeren, insbesondere von Formmassen hieraus, und von Lacküberzügen verwendet.

Ein weiteres bevorzugtes Einsatzgebiet ist die Stabilisierung von Polyethylen niedriger und hoher Dichte, sowie von Polypropylen und Polyamid, beispielsweise auch von Fasern hieraus.

Die Erfindung wird durch die Beispiele näher erläutert, ohne sie dadurch einzuschränken.

### Beispiele

### 1. Herstellung einer erfindungsgemäßen Mischung

500 g Poly-{3-(eicosyltetracosyl)-1-[2,2,6,6-tetramethylpiperidin-4-yl]-pyrrolidin-2,5-dion} (CAS Nr. 152261-33-1; HALS Ia) wurden durch Erwärmen geschmolzen und mit der gleichen Menge N,N'-Bisformyl-N,N'-bis-(2,2,6,6-tetramethyl-4-piperidinyl)hexamethylendiamin (CAS Nr. 124172-53-8; HALS IIa) versetzt. Nach der Homogenisierung wurden aus der heißen Mischung Pastillen geformt, die beim Erkalten erstarrten.

### 2. Lagerfähigkeit der erfindungsgemäßen Mischung

Um die Verbackungsneigung zu testen, wurde Poly-{3-(eicosyltetracosyl)-1-[2,2,6,6-tetramethylpiperidin-4-yl]-pyrrolidin-2,5-dion} (CAS Nr. 152261-33-1; HALS Ia) sowie die in Beispiel 1 hergestellte Mischung separat in Kunststofftütchen bei 40°C gelagert. Nach 20 h wurden die Proben visuell begutachtet.

| | **Probe** | **Visuelle Begutachtung nach 20 h bei 40°C** |
|---|---|---|
| 1 | HALS Ia | Vollständige Verbackung |
| 2 | Mischung aus HALS Ia und HALS IIa | Keine Verbackung (1:1) |

Das Beispiel zeigt, dass die oligomere Komponente A schon unter milden Bedingungen zur Verbackung neigt. Im Gegensatz dazu verbackt die erfindungsgemäße Mischung nicht.

### 3. Synergistische Wirkung der Mischung gegenüber den Einzelkomponenten

### 3a) Bewitterung von eingefärbten Polypropylen-(PP)Plättchen

Rot eingefärbte PP-Plättchen mit verschiedenen HALS-Stabilisatoren wurden hergestellt und anschließend gemäß DIN EN ISO 4892-2 bewittert. Die Schlagbiegefestigkeit wurde gemäß DIN 53453 gemessen. Die Ergebnisse sind in der folgenden Tabelle dargestellt:

| | **Stabilisierung^{*)}** | **Schlagbiegefestigkeit [mJ/mm²]** | | | |
|---|---|---|---|---|---|
| | | **0 h** | **2000 h** | **3000 h** | **4000 h** |
| 1 | 3000 ppm HALS IIa | 45 | 45 | 45 | 45 |
| 2 | 1250 ppm HALS Ia | 45 | 45 | 46 | 45 |
| | 1250 ppm HALS IIa | | | | |
| 3 | 1250 ppm HALS IVa | 44 | 45 | 42 | 40 |
| | 1250 ppm HALS IVb | | | | |

| | | | | | |
|---|---|---|---|---|---|
| *) HALS IVa: Polymer aus 2,2,4,4,-Tetramethyl-7-oxa-3,20-diaza-dispiro[5.1.11.2]-heneicosan-21-on und Epichlorhydrin (CAS Nr. 202483-55-4); HALS IVb: 2,2,4,4,-Tetramethyl-7-oxa-3,20-diaza-dispiro[5.1.11.2]-heneicosan-21-on (CAS Nr. 64338-16-5). | | | | | |

Anhand der Ergebnisse ist zu erkennen, dass eine geringere Menge der erfindungsgemäßen Mischung aus HALS Ia und HALS IIa (Ifd. Nr. 2) nötig ist, um die gleiche Stabilisierungswirkung zu erzielen wie mit der niedermolekularen HALS-Verbindung IIa alleine (Ifd. Nr. 1). Anhand der dritten Reihe ist zu sehen, dass andere Mischungen aus nieder- und hochmolekularen HALS-Verbindungen eine deutlich schlechtere Stabilisierung bewirken als die erfindungsgemäße Stabilisatormischung.

### 3b) Stabilisierung von Polypropylen-Bändchen

Gereckte (1:5) Bändchen aus Polypropylen mit verschiedenen Stabilisatoren wurden hergestellt und gemäß DIN EN ISO 4892-2 bewittert. Die Reißfestigkeit der Proben wurde in regelmäßigen Abständen getestet. Sobald die verbleibende Reißfestigkeit kleiner oder gleich 50 % der anfänglichen Reißfestigkeit war, galt die Probe als zerstört:

| | **Stabilisierung^{*)}** | **Stunden bis zur Zerstörung** |
|---|---|---|
| 1 | 1000 ppm HALS Ia | 2800 |
| 2 | 500 ppm HALS Ia | |
| | 500 ppm HALS IIa | 3200 |
| 3 | 1000 ppm HALS V | 2200 |

| | | |
|---|---|---|
| *) HALS Ia: Poly-{3-(eicosyl-tetracosyl)-1-[2,2,6,6-tetramethylpiperidin-4-yl]-pyrrolidin-2,5-dion} (CAS Nr. 152261-33-1); HALS IIa: N,N'-Bisformyl-N,N'-bis-(2,2,6,6-tetramethyl-4-piperidinyl)- hexamethylendiamin (CAS Nr. 124172-53-8); HALS V: Polymer von 4-Hydroxy-2,2,6,6-tetramethyl-1-piperidinylethanol- und Butandisäuredimethylester | | |

## Patentansprüche

1. Mischung, enthaltend
A. mindestens eine oligomere Verbindung, enthaltend Wiederholeinheiten der Formel (I), 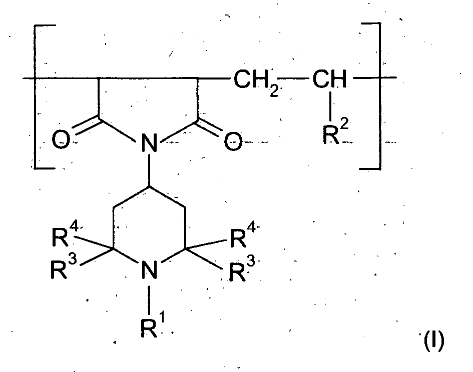 worin die Symbole folgende Bedeutungen haben,
R¹ ist Wasserstoff, C₁- bis C₆-Alkyl, Formyl, C₂- bis C₆-Alkanoyl, C₁- bis C₁₂-Alkoxy, C₅- bis C₆-Cycloalkoxy, Cyanomethyl, 2-Hydroxyethyl, Benzyl oder ein Rest der Formel -CR'=CH-CO-OR", wobei
R' Wasserstoff, C₁- bis C₆-Alkyl oder einen Rest der Formel -CO-OR" bedeutet und
R" C₁- bis C₁₈-Alkyl, C₅- bis C₈-Cycloalkyl, C₇- bis C₁₈-Aralkyl, Phenyl oder Tolyl bedeutet;
R² ist eine Mischung aus C₁₄- bis C₂₈-Alkylgruppen, wobei zwei dieser Alkylgruppen, die sich um nicht mehr als zwei C-Atome unterscheiden dürfen, jeweils mindestens 30 % dieser Mischung ausmachen;
R³, R⁴ sind unabhängig voneinander C₁- bis C₆-Alkyl;
und
B. mindestens eine Verbindung der Formel (II) oder (III), 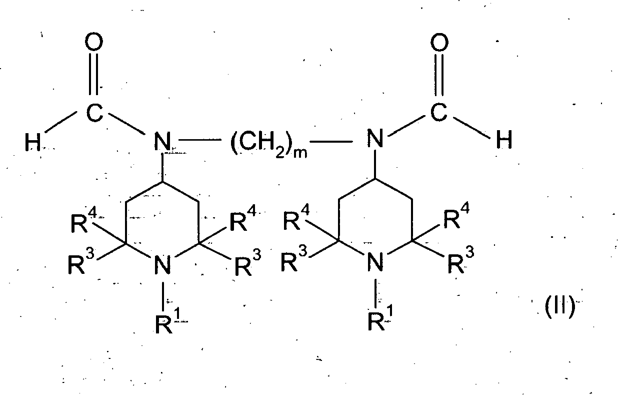 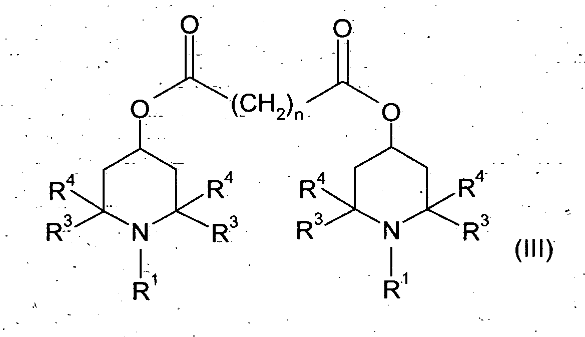 wobei die Symbole und Indizes folgende Bedeutungen haben:
n, m sind unabhängig voneinander eine natürliche Zahl von 2 bis 22 und
R¹, R³, R⁴ haben unabhängig voneinander die in Formel (I) angegebenen Bedeutungen.

2. Mischung gemäß Anspruch 1, wobei die Symbole und Indizes in den Formeln (I) bis (III) folgende Bedeutungen haben:
R¹ ist H, C₁-C₆-Alkyl, Formyl, Acyl, C₁-C₆-Alkyl oder Benzyl;
R² ist eine Mischung aus C₁₆-C₂₄-Alkylgruppen;
R³ und R⁴ sind Methyl und
m und n sind eine natürliche Zahl von 6 bis 8.

3. Mischung gemäß Anspruch 1 oder 2, wobei die Komponente A ein Oligomer der Formel (la) ist 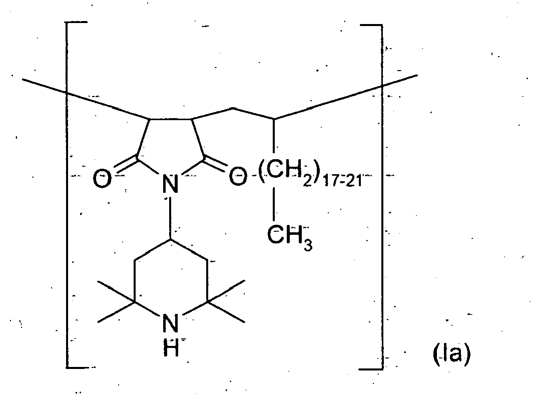 und die Komponente B ausgewählt ist aus der Gruppe der Verbindungen (IIa), (IIIa) und (IIIb): 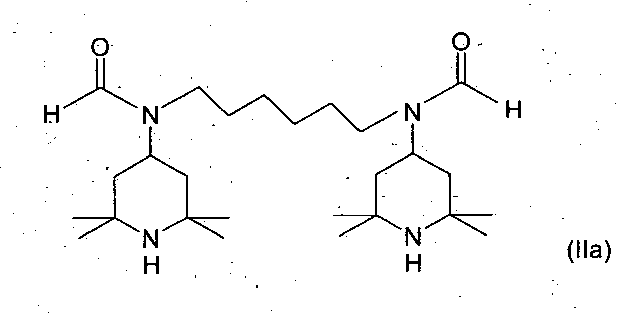 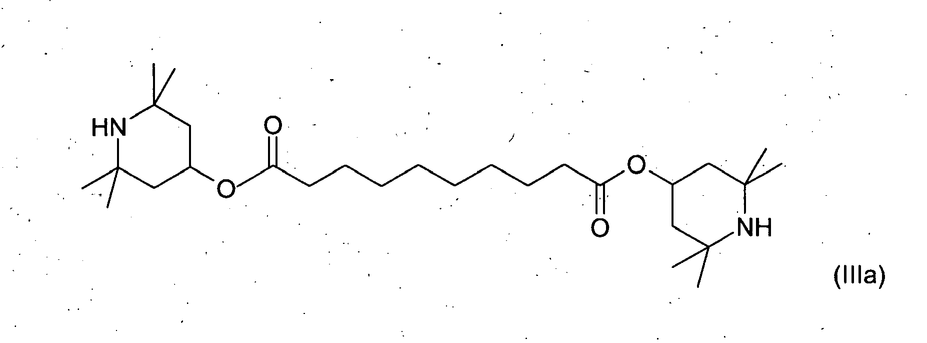 und

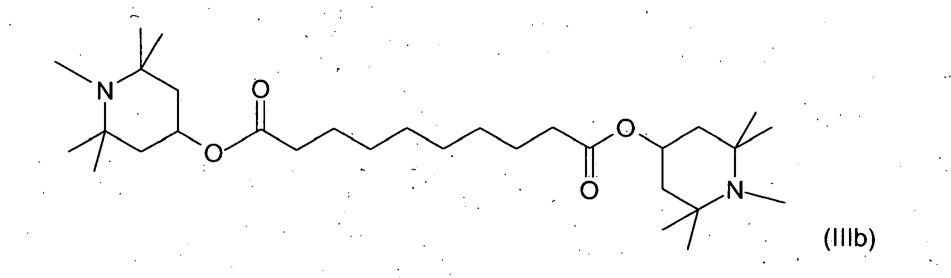

4. Verfahren zur Herstellung einer Mischung gemäß einem der Ansprüche 1 bis 3, wobei man
a) die Komponente (B) einer Schmelze der Komponente (A) zusetzt,
b) homogenisiert und
c) die erhaltene Mischung in eine gewünschte Form bringt und erkalten lässt.

5. Gegen die Einwirkung von Licht, Sauerstoff und Wärme stabilisiertes organisches Material, enthaltend 0,01 bis 5 Gew.-%, bezogen auf die Menge des organischen Materials, einer Mischung gemäß einem der Ansprüche 1 bis 3.

6. Gegen die Einwirkung von Licht, Sauerstoff und Wärme stabilisierte Kunststoffe und Lacke, enthaltend 0,01 bis 5 Gew.-%, bezogen auf die Menge der Kunststoffe bzw. Lacke, einer Mischung gemäß einem der Ansprüche 1 bis 3.

7. Verwendung von Mischungen gemäß einem der Ansprüche 1 bis 3 als Lichtschutzmittel und Stabilisatoren für organisches Material.

8. Verwendung gemäß Anspruch 7 als Lichtschutzmittel und Stabilisatoren für Kunststoffe und Lacke.

9. Verfahren zum Stabilisieren von organischem Material gegen die Einwirkung von Licht, Sauerstoff und Wärme, **dadurch gekennzeichnet, dass** man hierzu eine Mischung gemäß einem der Ansprüche 1 bis 3 verwendet.

10. Verfahren zum Stabilisieren von Kunststoffen und Lacken gegen die Einwirkung von Licht, Sauerstoff und Wärme, **dadurch gekenntzeichnet, dass** man hierzu eine Mischung gemäß einem der Ansprüche 1 bis 3 verwendet.

## Claims

1. A mixture, comprising
A. at least one oligomeric compound, comprising repeat units of the formula (I), 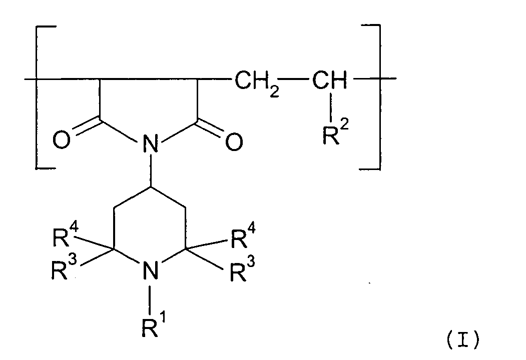 in which the meanings of the symbols are as follows:
R¹ is hydrogen, C₁-C₆-alkyl, formyl, C₂-C₆- alkanoyl, C₁-C₁₂-alkoxy, C₅-C₆-cycloalkoxy, cyanomethyl, 2-hydroxyethyl, benzyl or a radical of the formula -CR'=CH-CO-OR'', where
R' is hydrogen, C₁-C₆-alkyl or a radical of the formula -CO-OR'', and
R'' is C₁-C₁₈-alkyl, C₅-C₈-cycloalkyl, C₇-C₁₈- aralkyl, phenyl, or tolyl;
R² is a mixture composed of C₁₄-C₂₈-alkyl groups, where two of these alkyl groups whose number of carbon atoms is not permitted to differ by more than two respectively make up at least 30% of this mixture;
R³ and R⁴, independently of one another, are C₁-C₆- alkyl;
and
B. at least one compound of the formula (II) or (III) 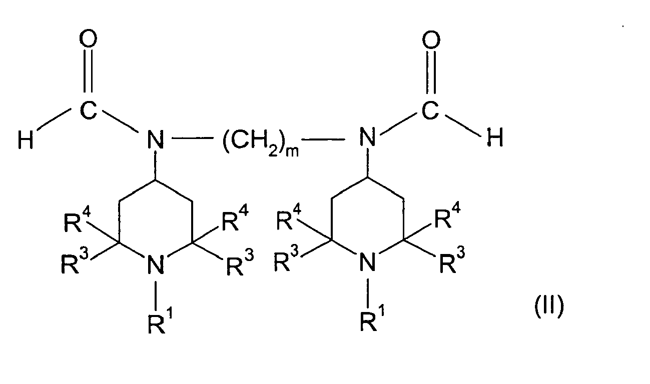 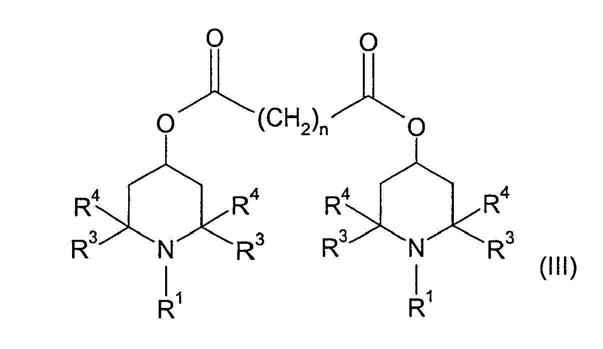 where the meanings of the symbols and indices are as follows:
n and m, independently of one another, are a natural number from 2 to 22, and
R¹, R³ and R⁴, independently of one another, have the meanings given in formula (I).

2. The mixture according to claim 1, where the meanings of the symbols and indices in the formulae (I) to (III) are as follows:
R¹ is H, C₁-C₆-alkyl, formyl, acyl, C₁-C₆- alkyl or benzyl;
R² is a mixture composed of C₁₆-C₂₄-alkyl groups;
R³ and R⁴ are methyl, and
m and n are a natural number from 6 to 8.

3. The mixture according to claim 1 or 2, where component A is an oligomer of the formula (Ia) 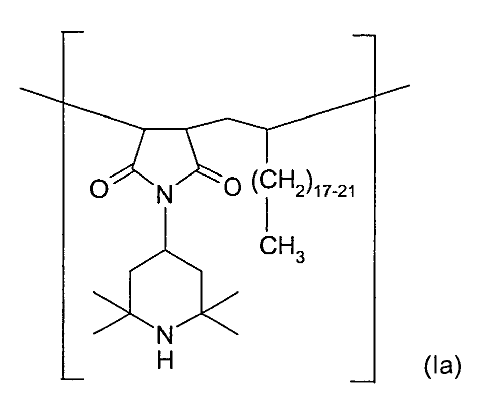 and component B has been selected from the group of the compounds (IIa), (IIIa), and (IIIb): 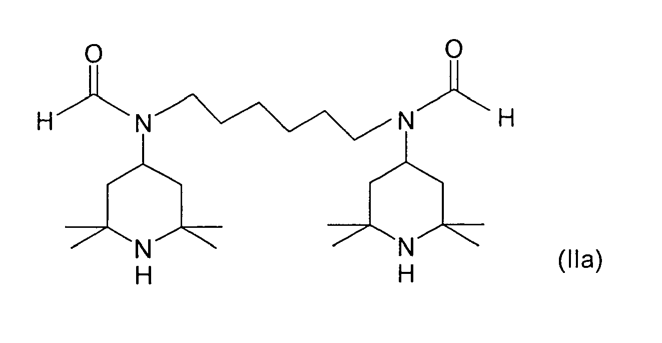 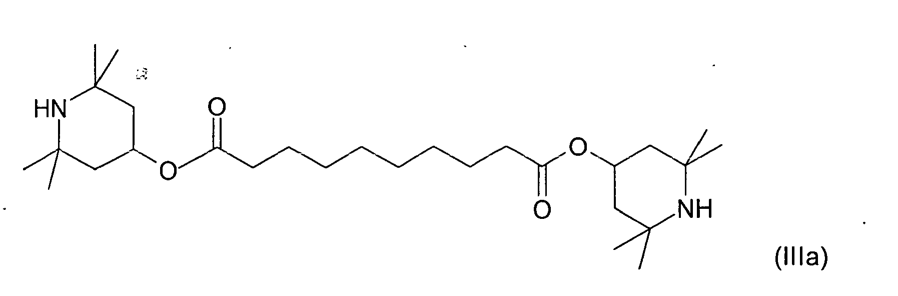 and

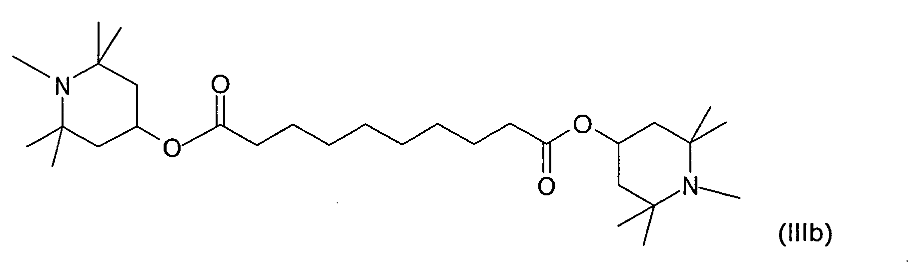

4. A process for preparation of a mixture according to any of claims 1 to 3,
where
a) component (B) is added to a melt of component (A),
b) the mixture is homogenized, and
c) the resultant mixture is converted to a desired form and allowed to cool.

5. An organic material stabilized with respect to exposure to light, oxygen, and heat, comprising from 0.01 to 5% by weight, based on the amount of the organic material, of a mixture according to any of claims 1 to 3.

6. A plastic or paint stabilized with respect to exposure to light, oxygen, and heat, comprising from 0.01 to 5% by weight, based on the amount of the plastic and, respectively, paint, of a mixture according to any of claims 1 to 3.

7. The use of mixtures according to any of claims 1 to 3 as light stabilizers and other stabilizers for organic material.

8. The use according to claim 7 as light stabilizers and other stabilizers for plastics and paints.

9. A method for stabilization of organic material with respect to exposure to light, oxygen, and heat, which comprises using for this purpose a mixture according to any of claims 1 to 3.

10. A method for stabilization of plastics and paints with respect to exposure to light, oxygen, and heat, which comprises using for this purpose a mixture according to any of claims 1 to 3.

## Revendications

1. Mélange, contenant
A. au moins un composé oligomère, contenant des unités de répétition de formule (I), 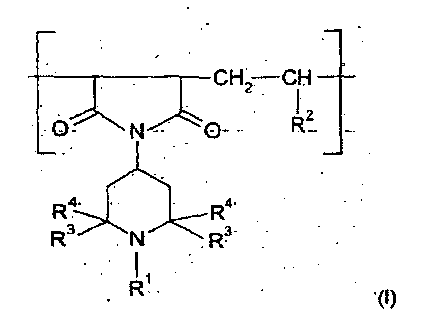 dans laquelle les symboles ont les significations suivantes,
R¹ représente l'hydrogène, un alkyle en C₁ à C₆, un formyle, un alcanoyle en C₂ à C₆, un alcoxy en C₁ à C₁₂, un cycloalcoxy en C₅ à C₆, un cyanométhyle, un 2-hydroxyéthyle, un benzyle ou un radical de formule -CR'=CH-CO-OR'', dans lequel
R' représente l'hydrogène, un alkyle en C₁ à C₆ ou un radical de formule -CO-OR'' et
R'' représente un alkyle en C₁ à C₁₈, un cycloalkyle en C₅ à C₈, un aralkyle en C₇ à C₁₈, un phényle ou un tolyle ;
R² représente un mélange de groupes alkyles en C₁₄ à C₂₈, deux de ces groupes alkyles, qui peuvent différer au plus de deux atomes C, représentant chacun au moins 30 % de ce mélange ;
R³, R⁴ représentent indépendamment l'un de l'autre un alkyle en C₁ à C₆ ;
et
B. au moins un composé de formule (II) ou (III), 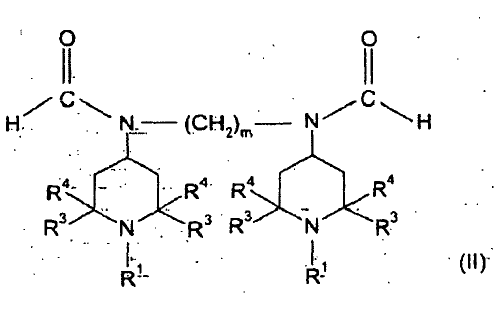 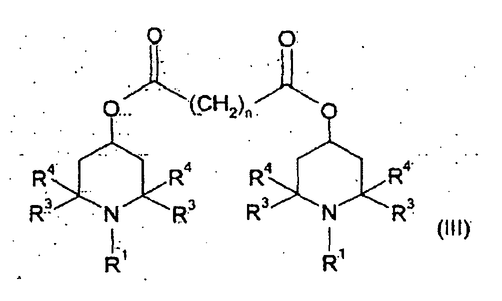 dans lesquelles les symboles et les indices ont les significations suivantes :
n, m représentent indépendamment l'un de l'autre un nombre naturel de 2 à 22 et
R¹, R³, R⁴ ont indépendamment les uns des autres les significations données pour la formule (I).

2. Mélange selon la revendication 1, dans lequel les symboles et les indices des formules (I) à (III) ont les significations suivantes :
R¹ représente H, un alkyle en C₁ à C₆, un formyle, un acyle, un alkyle en C₁ à C₆ ou un benzyle ;
R² représente un mélange de groupes alkyles en C₁₆ à C₂₄ ;
R³ et R⁴ représentent un méthyle et
m et n représentent un nombre naturel de 6 à 8.

3. Mélange selon la revendication 1 ou 2, dans lequel le composant A est un oligomère de formule (Ia) 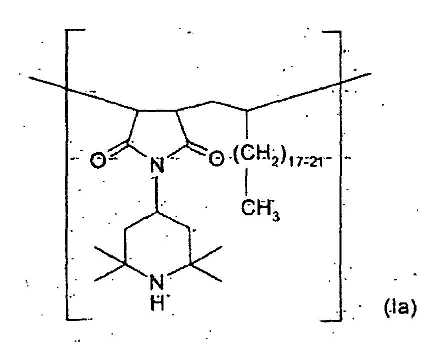 et le composant B est choisi dans le groupe des composés (IIa), (IIIa) et (IIIb) : 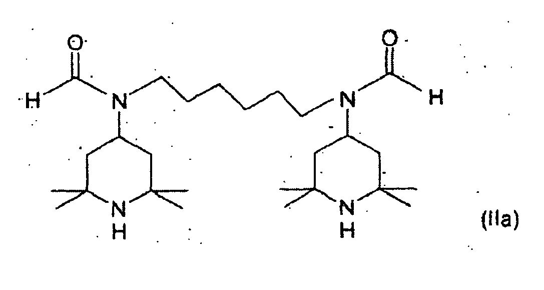 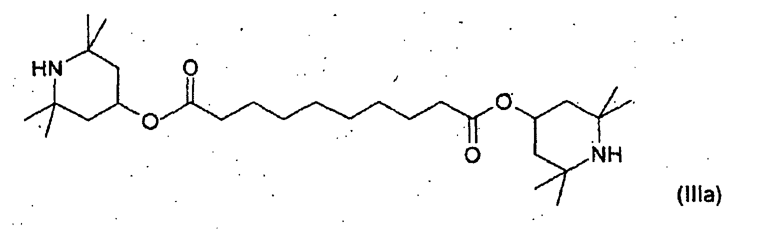 et

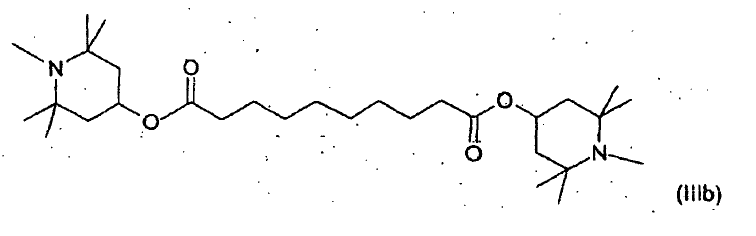

4. Procédé de fabrication d'un mélange selon l'une quelconque des revendications 1 à 3, dans lequel
a) le composant (B) est ajouté à une masse fondue du composant (A),
b) homogénéisé et
c) le mélange obtenu est mis sous une forme souhaitée et laissé refroidir.

5. Matériau organique stabilisé envers l'action de la lumière, de l'oxygène et de la chaleur, contenant 0,01 à 5 % en poids, par rapport à la quantité du matériau organique, d'un mélange selon l'une quelconque des revendications 1 à 3.

6. Plastiques et laques stabilisés envers l'action de la lumière, de l'oxygène et de la chaleur, contenant 0,01 à 5 % en poids, par rapport à la quantité des plastiques ou des laques, d'un mélange selon l'une quelconque des revendications 1 à 3.

7. Utilisation de mélanges selon l'une quelconque des revendications 1 à 3 en tant que photoprotecteurs et stabilisateurs pour un matériau organique.

8. Utilisation selon la revendication 7 en tant que photoprotecteurs et stabilisateurs pour des plastiques et des laques.

9. Procédé de stabilisation d'un matériau organique envers l'action de la lumière, de l'oxygène et de la chaleur, **caractérisé en ce qu'**un mélange selon l'une quelconque des revendications 1 à 3 est pour cela utilisé.

10. Procédé de stabilisation de plastiques et de laques envers l'action de la lumière, de l'oxygène et de la chaleur, **caractérisé en ce qu'**un mélange selon l'une quelconque des revendications 1 à 3 est pour cela utilisé.
